(19) Europäisches Patentamt
European Patent Office
Office européen des brevets

(11) Veröffentlichungsnummer: **0 037 004**
**B1**

# (12) EUROPÄISCHE PATENTSCHRIFT

(45) Veröffentlichungstag der Patentschrift: **08.06.83**

(51) Int. Cl.³: **C 07 C 45/37**, C 07 C 49/185, B 01 J 23/24, B 01 J 23/28, B 01 J 23/68, B 01 J 23/88

(21) Anmeldenummer: **81101975.1**

(22) Anmeldetag: **17.03.81**

## (54) Verfahren zur Herstellung von Methylglyoxal.

(30) Priorität: **28.03.80 DE 3012004**

(43) Veröffentlichungstag der Anmeldung: **07.10.81 Patentblatt 81/40**

(45) Bekanntmachung des Hinweises auf die Patenterteilung: **08.06.83 Patentblatt 83/23**

(84) Benannte Vertragsstaaten: **BE DE FR GB NL**

(56) Entgegenhaltungen: **DE-C-918 746**
**US-A-2 339 282**

(73) Patentinhaber: **HOECHST AKTIENGESELLSCHAFT, Postfach 80 03 20, D-6230 Frankfurt/Main 80 (DE)**

(72) Erfinder: **Baltes, Herbert, Dr., Johannesallee 24, D-6230 Frankfurt am Main 80 (DE)**
Erfinder: **Leupold, Ernst Ingo, Dr., Am Zäunefeld 15, D-6392 Neu-Anspach (DE)**

**EP 0 037 004 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Methylglyoxal

Die Erfindung betrifft ein Verfahren zur Herstellung von Methylglyoxal durch Oxidation von Propylenglykol-1.2 in der Gasphase an einem heterogenen Katalysator.

Aus US–PS 2 051 266 ist bekannt, dass sich ein Glykol der Formel I mit molekularem Sauerstoff in unterstöchiometrischer Menge katalytisch zu der entsprechenden Dicarbonylverbindung der Formel II oxidieren lässt:

$$\begin{array}{l} \mathrm{OH\ \ OH} \qquad\qquad\qquad \mathrm{O\ \ O} \\ |\qquad | \qquad\qquad\qquad\quad \|\ \ \| \\ \mathrm{CH_2{-}CH{-}R + O_2 \longrightarrow HC{-}C{-}R + 2H_2O} \\ \qquad \mathrm{I} \qquad\qquad\qquad\qquad \mathrm{II} \end{array}$$

Der Sauerstoff-Unterschuss soll verhindern, dass die Dicarbonylverbindung II in Folgereaktionen weiteroxidiert wird, z. B. zu der entsprechenden Carbonsäure oder unter Spaltung von C-C-Bindungen zu Formaldehyd oder Kohlenoxiden. Aufgrund des Sauerstoff-Unterschusses entstehen jedoch auch Produkte mit niedrigerer Oxidationszahl als der erwünschten, z.B. Hydroxycarbonylverbindungen. Ausserdem müssen ein unvollständiger Umsatz des Ausgangsproduktes I und die damit verbundenen Ausbeuteverluste bzw. Trennprobleme in Kauf genommen werden. Nicht-umgesetztes Glykol I reagiert ausserdem mit der gebildeten Dicarbonylverbindung II zu hochsiedenden, stabilen Acetalen oder anderen hochmolekularen Kondensationsprodukten. Auch dadurch werden grosse Ausbeuteverluste und beträchtliche Trennprobleme verursacht (US–PS 2 339 346).

Als katalytisch aktive Metalle werden in der genannten US–PS 2 051 266 vor allem die Nebengruppenelemente der vierten Periode (Titan bis Zink), insbesondere Kupfer, genannt.

Aufgrund der erwähnten Nachteile des Verfahrens gemäss US–PS 2 051 266 bei der Herstellung von Methylglyoxal aus Propylenglykol-1.2 wurden mehrere Modifikationen vorgeschlagen:

Gemäss US–PS 2 339 282 und US–PS 2 339 346 kann man mit einem Molybdän und Titan enthaltenden Katalysator und einem Sauerstoff-Überschuss arbeiten, aber vorzugsweise wird ein Kupferkatalysator verwendet, der kein Molybdän enthält und auch mit diesem Katalysator sind die Ergebnisse unbefriedigend.

Gemäss US–PS 2 339 346 werden ausserdem geringe Mengen Halogene oder organische Halogenverbindungen zugesetzt. Es ist jedoch offensichtlich, dass dieser Zusatz Werkstoffprobleme verursacht und die Aufarbeitung kompliziert.

Bei dem Verfahren gemäss DE–PS 857 359 wird 10 bis 50% der theoretisch erforderlichen Sauerstoffmenge eingesetzt. Bei einer Methylglyoxal-Ausbeute von nur 50% erzielt man einen unvollständigen Umsatz von Propylenglykol-1.2 mit allen oben geschilderten Nachteilen.

In DE–PS 1 923 048 wird ein Verfahren zur Oxidation von Propylenglykol-1.2 zu Methylglyoxal beschrieben, bei dem ein Katalysator verwendet wird, der Kupfer in Kombination mit Zinn, Phosphor, Arsen, Antimon und/oder Wismut enthält. Molybdän wird nicht erwähnt. Sauerstoff wird vorzugsweise im Überschuss eingesetzt. Nach diesem Verfahren erhält man bei einem Umsatz von 93% eine Ausbeute an Methylglyoxal von ca. 70%. Sowohl der unvollständige Umsatz wie auch das Auftreten von 1-Hydroxypropanon sind jedoch unbefriedigend und verhindern eine einfache Aufarbeitung.

Gemäss den tschechoslowakischen Patentschriften 140 414 und 119 683 wird an Silber-Katalysatoren bei Temperaturen von 530 °C bis 600 °C ein Propylenglykol-1.2-Umsatz von maximal 53% erzielt. Abgesehen von diesem unbefriedigenden Umsatz und den daraus resultierenden Problemen bringt die relativ hohe Reaktionstemperatur apparative Probleme mit sich.

Alle diese Verfahren sind im Hinblick auf hohe Ausbeuten und einen einfachen, wirtschaftlichen Betrieb unbefriedigend.

Der vorliegenden Erfindung liegt daher die Aufgabe zugrunde, ein Verfahren zu entwickeln, dass die bisherigen Probleme:

– Auftreten von Produkten mit einer niedrigen Oxidationsstufe als der gewünschten.

– Folgereaktionen des gebildeten Methylglyoxals mit nichtumgesetztem Propylenglykol-1.2

– Verunreinigung durch Hilfsstoffe

überwindet. Diese Aufgabe wird durch Verwendung eines spezifischen Katalysatorsystems in Verbindung mit einem Sauerstoffüberschuss gelöst.

Gegenstand der vorliegenden Erfindung ist ein Verfahren zur Herstellung von Methylglyoxal durch Oxidation von Propylenglykol-1.2 in der Gasphase an einem heterogenen Molybdän-Katalysator, das dadurch gekennzeichnet ist, dass mit Sauerstoff-Überschuss gearbeitet wird und dass der Katalysator zusätzlich mindestens eines der folgenden Elemente enthält: Vanadin, Silber, Kupfer, Eisen, Wolfram, Zinn, Zink, Alkalimetalle, Erdalkalimetalle.

Dieses Verfahren zeichnet sich gegenüber den bisher beschriebenen durch wesentlich höhere Ausbeuten und durch einen nahezu quantitiven Propylenglykol-1.2-Umsatz aus. Cyclische Acetale (Dioxolane) aus nicht-umgesetzten Propylenglykol-1.2 und gebildetem Methylglyoxal treten daher praktisch nicht auf. Produkte mit einer niedrigeren Oxidationsstufe werden ebenfalls nicht gebildet, und Hilfsstoffe werden nicht benutzt. Die Aufarbeitung vereinfacht sich dadurch beträchtlich.

Bei dem erfindungsgemässen Verfahren wird Propylenglykol-1.2 allein oder als wässrige Lösung verdampft und gemeinsam mit Sauerstoff oder einem sauerstoffhaltigen Gas, insbesondere Luft, über den Katalysator geleitet.

Es hat sich als vorteilhaft erwiesen, zusätzlich noch ein unter den Reaktionsbedingungen inertes Trägergas zuzumischen. Als Trägergase kommen

z.B. Stickstoff und Edelgase in Frage, aber auch niedere Kohlenwasserstoffe wie Methan, Ethan oder Propan. Falls man Luft als sauerstoffhaltiges Gas verwendet, erfüllt diese gleichzeitig die Funktion des Trägergases.

Bei dem erfindungsgemässen Verfahren setzt man pro Mol Propylenglykol-1.2 i.allg. folgende Mengen an Zusatzstoffen ein:

Wasser:
0 bis 15 Mol, vorzugsweise 0 bis 5 Mol,
Sauerstoff:
1.2 bis 10 Mol, vorzugsweise 1.5 bis 5 Mol,
Trägergas:
0 bis 80 Mol, vorzugsweise 10 bis 80 Mol, insbesondere 40 bis 60 Mol.

Auch ausserhalb dieser Grenzen werden noch zufriedenstellende Ergebnisse erzielt.

Der Katalysator enthält neben Molybdän noch mindestens eines der folgenden Elemente: Vanadin, Silber, Kupfer, Eisen, Wolfram, Zinn, Zink, Alkalimetalle, Erdalkalimetalle. Vorzugsweise verwendet man Vanadin, Silber, Kupfer, Natrium, Kalium, Magnesium, Barium, insbesondere Vanadin, Silber, Natrium, Kalium.

Als ganz besonders vorteilhaft haben sich Katalysatoren erwiesen, die Molybdän, Vanadin und mindestens eines der folgenden Elemente enthalten: Silber, Natrium, Kalium, insbesondere Silber.

Die genannten Elemente werden entweder in metallischer Form oder in Form ihrer Verbindungen z.B. als Oxide, Nitrate, Acetate, Acetylacetonate, Oxalate, Citrate oder Halogenide in die Reaktionszone eingebracht.

Es hat sich als vorteilhaft erwiesen, zur Aktivierung des Katalysators vor Beginn der Reaktion ein oxidierendes Gas, insbesondere Sauerstoff oder Luft, bei Temperaturen von 100 °C bis 800 °C, insbesondere von 300 °C bis 600 °C, über den Katalysator zu leiten.

Die katalytisch aktiven Elemente werden vorzugsweise auf Trägermaterialien aufgezogen. Als Träger eignen sich vor allem Silicate, Aluminiumoxide, Aluminiumsilikate, Bimsstein oder Kohlen. Insbesondere verwendet man Silikate, Aluminiumoxide oder Aluminiumsilikate. Besonders vorteilhaft sind oberflächenarme Aluminiumsilikate und Aluminiumoxide, insbesondere α-Aluminiumoxid, mit einer BET-Oberfläche von weniger als 20 $m^2/g$.

Die Gesamtmenge an katalytisch aktiven Elementen kann in weiten Grenzen schwanken. Im allgemeinen beträgt sie 0.01 bis 50 Gew.-%, vorzugsweise 0.5 bis 20 Gew.-%, bezogen auf die Gesamtmasse des Trägerkatalysators. Dabei beträgt das Gewichts-Verhältnis von Molybdän zur Gesamtmenge der restlichen auf den Träger aufgebrachten katalytisch aktiven Elemente i.allg. 1:0.01 bis 1:10, vorzugsweise 1:0.1 bis 1:2.

Die katalytisch aktiven Komponenten werden zweckmässig in Form einer Lösung auf den Träger aufgebracht; dann wird das Lösemittel abgedampft und der Katalysator getrocknet. Als Lösemittel verwendet man i.allg. Wasser, Salzsäure, Salpetersäure, Alkalilaugen oder wässrige Ammoniaklösung, vorzugsweise Wasser oder wässrige Ammoniaklösung.

Die aktiven Komponenten können jedoch auch ohne Träger eingesetzt werden.

Das erfindungsgemässe Verfahren wird i.allg. bei Temperaturen zwischen 100 °C und 600 °C, vorzugsweise zwischen 200 °C und 450 °C durchgeführt.

Die Verweilzeit liegt vorzugsweise zwischen 0.1 und 10 Sekunden, insbesondere jedoch zwischen 0.1 und 1 Sekunde. Auch ausserhalb dieser Grenzen enthält man noch befriedigende Ergebnisse.

Das erfindungsgemässe Verfahren wird bevorzugt bei Normaldruck durchgeführt, jedoch können auch verminderte oder erhöhte Drucke angewendet werden (0.01 bis 100 bar).

Eine geeignete Ausführungsform ist z.B. die folgende: Das Propylenglykol-1.2 bzw. ein Gemisch aus Propylenglykol-1.2 und Wasser wird aus einer Dosiervorrichtung in eine Verdampfungszone und das entstandene Gas dann durch ein von aussen beheiztes und mit dem Katalysator gefülltes Reaktionsrohr geleitet. In der Verdampfungszone erfolgt die Vermischung mit dem Sauerstoff oder dem sauerstoffhaltigen Gas und ggf. mit dem Trägergas; es hat sich als vorteilhaft erwiesen, diese Gase vor der Vermischung auf die Reaktionstemperatur aufzuheizen. Die Reaktionsprodukte werden nach Verlassen des Reaktors zur Abtrennung der kondensierbaren Anteile gekühlt. Aus dem Kondensat lässt sich das Methylglyoxal nach üblichen Methoden, z.B. gemäss DE-PS 1 914 038 durch Schnelldestillation oder gemäss US-PS 2 866 823 als Dimethylacetal isolieren.

Wenn ein Gemisch aus Propylenglykol-1.2 und Wasser eingesetzt wird, ist das Kondensat eine wässrige Lösung, die für viele Einsatzgebiete auch direkt verwendet werden kann, z.B. zur Herstellung von Acetalen des Methylglyoxals (US-PS 2 421 559).

Methylglyoxal und die Acetale des Methylglyoxals eignen sich aufgrund ihrer hohen Reaktivität als Zwischenprodukte für die Herstellung zahlreicher chemischer Verbindungen, wie z.B. hochwirksamer Insektizide vom Allethrin-Typ (H.J. Sanders und A.W. Taff, Ind. Eng. Chem. 46, 414-426 [1954]).

Beispiel 1

12 ml/h einer 50%igen wässrigen Propylenglykol-1.2-Lösung werden mit Hilfe einer Kolbenspritze über eine Verdampfungszone in einen senkrecht angeordneten Glasreaktor von 150 mm Länge und 20 mm Durchmesser eingeleitet. Dabei werden der Verdampfungszone gleichzeitig 56 Nl/h Stickstoff und 7.4 Nl/h Sauerstoff zugeführt, die beide vorher auf 350 °C aufgeheizt worden sind.

Der Reaktor wird von aussen ebenfalls auf 350 °C geheizt und ist mit 15 ml eines Aluminiumsilikat-Katalysators gefüllt, welcher 4.9 Gew.-% Molybdän und 5.1 Gew.-% Vanadin enthält und eine BET-Oberfläche von ca. 1 $m^2/g$ besitzt.

Zur Herstellung des Katalysators löst man die berechnete Menge Ammoniummolybdat $(NH_4)_2$

$MoO_4$ und Ammoniumvanadat ($NH_4VO_3$) in konzentrierter Ammoniak-Lösung, tränkt den Katalysatorträger mit dieser Lösung und dampft das Lösemittel auf dem Dampfbad ab. Der Katalysator wird anschliessend bei 110 °C getrocknet und dann im Reaktor in einem Gasstrom aus 56 Nl/h Stickstoff und 3 Nl/h Sauerstoff 3 Stunden lang auf 400 °C erhitzt.

Die Temperatur im Innern des Reaktors wird mit Hilfe eines Thermoelementes gemessen. Die Reaktionsprodukte werden in einer Kühlfalle bei –70 °C kondensiert.

Nach einer Anlaufzeit von 1 Stunde zur Einstellung konstanter Betriebsbedingungen wird der eigentliche Katalysatortest über einen Zeitraum von 2 Stunden durchgeführt. Das Kondensat wird flüssigkeitschromatographisch analysiert.

Als Ergebnis eines zweistündigen Versuches werden 102 mMol Methylglyoxal gefunden, entsprechend einer Ausbeute von 63% . Der Propylenglykol-1.2-Umsatz beträgt 98.7%. Die Ausbeute an Dioxolanen, bezogen auf eingesetztes Propylenglykol-1.2, ist kleiner 1%.

Beispiel 2

Analog zu Beispiel 1 werden in die dort beschriebene Apparatur 12 ml/h 50%ige wässrige Propylenglykol-1.2-Lösung, 7.4 Nl/h Sauerstoff und 56 Nl/h Stickstoff eingeleitet. Im Reaktor befinden sich 15 ml eines Katalysators, der 6 Gew.-% Vanadinpentoxid, 3 Gew.-% Molybdäntrioxid und 0.2 Gew.-% Silber auf einem Alpha-Aluminium-Träger (BET-Oberfläche ca. 1 $m^2/g$) enthält und auf 350 °C geheizt wird.

Als Ergebnis eines zweistündigen Versuches erhält man 134 mMol Methylglyoxal, entsprechend einer Ausbeute von 83%. Der Propylenglykol-1.2-Umsatz ist grösser 99%.

Beispiel 3

Analog zu Beispiel 1 werden in dort beschriebene Apparatur 12 ml/h 50%ige wässrige Propylenglykol-1.2-Lösung, 3.7 Nl/h Sauerstoff und 56 Nl/h Stickstoff eingeleitet. Im Reaktor befinden sich 15 ml eines Aluminiumsilicat-Katalysators, der 3.8 Gew.-% Molybdän, 2.0 Gew.-% Vanadin, 4.0 Gew.-% Silber und 0.3 Gew.-% Kupfer auf einem Träger wie in Beispiel 1 enthält und auf 330 °C geheizt wird.

Als Ergebnis eines zweistündigen Versuches erhält man 123 mMol Methylglyoxal, entsprechend einer Ausbeute von 76%. Der Propylenglykol-1.2-Umsatz beträgt 96%.

Beispiel 4

Analog zu Beispiel 1 werden in die dort beschriebene Apparatur 6 ml/h Propylenglykol-1.2 und 18.5 Nl/h Luft eingeleitet. Im Reaktor befinden sich 15 ml eines Aluminiumsilikat-Katalysators, der 4.0 Gew.-% Molybdän, 3.0 Gew.-% Vanadin, 1.0 Gew.% Zink, 1.0 Gew. % Zinn und 1.0 Gew. % Wolfram auf einem Träger wie in Beispiel 1 enthält und auf 300 °C geheizt wird.

Als Ergebnis eines zweistündigen Versuches erhält man 113 mMol Methylglyoxal, entsprechend einer Ausbeute von 69%. Der Propylenglykol-1.2-Umsatz beträgt 94%.

Beispiel 5

Analog zu Beispiel 1 werden in die dort beschriebene Apparatur 12 ml/h 50%ige wässrige Propylenglykol-1.2-Lösung, 3.7 Nl/h Sauerstoff und 56 Nl/h Stickstoff eingeleitet. Im Reaktor befinden sich 15 ml eines Aluminiumsilicat-Katalysators, der 4.9 Gew. % Molybdän, 5.1 Gew. % Vanadin, 0.45 Gew. % Kalium, 0.45 Gew. % Magnesium und 0.23 Gew. % Eisen auf einem Träger wie in Beispiel 1 enthält und auf 350 °C geheizt wird.

Als Ergebnis eines zweistündigen Versuches erhält man 118 mMol Methylglyoxal, entsprechend einer Ausbeute von 73%. Der Propylenglykol-1.2-Umsatz geträgt 98.6%.

**Patentansprüche**

1. Verfahren zur Herstellung von Methylglyoxal durch Oxidation von Propylenglykol-1.2 in der Gasphase an einem heterogenen Molybdän-Katalysator, dadurch gekennzeichnet, dass mit Sauerstoff-Überschuss gearbeitet wird und dass der Katalysator zusätzlich mindestens eines der folgenden Elemente enthält: Vanadin, Silber, Kupfer, Eisen, Wolfram, Zinn, Zink, Alkalimetalle, Erdalkalimetalle.

2. Verfahren nach Anspruch 1, dadurch gekennzeichnet, dass der Katalysator Molybdän und mindestens eines der Elemente Vanadin, Silber Kupfer, Natrium, Kalium, Magnesium, Barium enthält.

3. Verfahren nach Anspruch 2, dadurch gekennzeichnet, dass der Katalysator Molybdän und mindestens eines der Elemente Vanadin, Silber, Natrium, Kalium enthält.

4. Verfahren nach Anspruch 1, dadurch gekennzeichnet, dass der Katalysator Molybdän, Vanadin und mindestens eines der Elemente Silber, Natrium, Kalium enthält.

5. Verfahren nach Anspruch 1, dadurch gekennzeichnet, dass der Katalysator Molybdän, Vanadin und Silber enthält.

**Revendications**

1. Procédé de préparation du méthylglyoxal par oxydation du propylène-glycol-1,2, en phase gazeuse, en présence d'un catalyseur hétérogène au molybdène, procédé caractérisé en ce qu'on opère avec une quantité d'oxygène inférieure à la quantité stoechiométrique et en ce que le catalyseur contient en outre au moins l'un des éléments suivants: vanadium, argent, cuivre, fer, tungstène, étain, zinc, métaux alcalins et métaux alcalinoterreux.

2. Procédé selon la revendication 1, caractérisé en ce que le catalyseur contient du molybdène et au moins l'un des éléments suivants: vanadium, argent, cuivre, sodium, potassium, magnésium et baryum.

3. Procédé selon la revendication 2, caractérisé en ce que le catalyseur contient du molybdène et au moins l'un des éléments; vanadium, argent, sodium et potassium.

4. Procédé selon la revendication 1, caractérisé en ce que le catalyseur contient du molybdène, du vanadium et au moins l'un des éléments: argent, sodium et potassium.

5. Procédé selon la revendication 1, caractérisé en ce que le catalyseur contient du molybdène, du vanadium et de l'argent.

**Claims**

1. A process for the manufacture of methyl glyoxal by oxidation of propylene glycol-1,2 in the gaseous phase on a heterogeneous molybdenum catalyst, characterized in that an excess of oxygen is used and the catalyst contains in addition at least one of the following elements: vanadium, silver, cooper, iron, tungsten, tin, zinc, alkali metals, alkaline earth metals.

2. The process of claim 1, characterized in that the catalyst contains molybdenum and at least one of the following elements: vanadium, silver, copper, sodium. potassium, magnesium, barium.

3. The process of claim 2, characterized in that the catalyst contains molybdenum and at least one of the elements vanadium, silver, sodium, potassium.

4. The process of claim 1, characterized in that the catalyst contains molybdenum, vanadium and at least one of the elements silver, sodium, potassium.

5. The process of claim 1, characterized in that the catalyst contains molybdenum, vanadium and silver.